# EUROPEAN PATENT APPLICATION

(11) **EP 4 464 788 A1**
(43) Date of publication of application: **20.11.2024**
(21) Application number: 23173977.2
(22) Date of filing: 17.05.2023
(51) Int. Cl.: C12Q 1/25, C12N 9/00, C12N 15/10

(54) **SYSTEM AND METHOD FOR CHECKING THE ABILITY OF A PROTEIN OF INTEREST TO ACT AS A SUBSTRATE FOR AN ENZYME**

(71) Applicant: Julius-Maximilians-Universität Würzburg, 97070 Würzburg (DE)
(72) Inventor: WOLF, Elmar, 97084 Wuerzburg (DE); ADHIKARI, Bikash, 97218 Gerbrunn (DE)
(74) Representative: Dr. Gassner & Partner mbB

(57) **Abstract**

The invention concerns a system for checking whether a protein of interest (POI) can act as a substrate, in particular a favorable substrate, for an enzyme selected from an E3-ubiquitin ligase, a SUMO E3 ligase, a deubiquitinating enzyme, a kinase or a phosphatase in a eukaryotic cellular environment, wherein the system comprises a first recombinant protein comprising an amino acid sequence of the enzyme and an amino acid sequence of a first binding protein of a dimerization system, a second recombinant protein comprising an amino acid sequence of the POI, an amino acid sequence of a reporter protein and an amino acid sequence of a second binding protein of the dimerization system and a ligand of the dimerization system.

## Description

The present invention concerns a system and a method for checking whether a protein of interest (POI) can act as a substrate for an enzyme and a use of such a system. The enzyme may in particular be an E3-ubiquitin ligase.

From WO 2021/180787 A1 a Proteolysis Targeting Chimera (PROTAC) for degradation of Aurora A-kinase is known. The PROTAC has the chemical structure AAB-L-E3B, wherein AAB is a binding unit for Aurora A-kinase, L is a connecting unit and E3B is a binding unit for E3-ubiquitin ligase Cereblon.

It has been recognized that not all PROTACs are able to combine a POI that shall be degraded with an E3-ubiquitin ligase such that the POI is degraded or effectively degraded in a eukaryotic cellular system as a consequence of ubiquitination by the E3-ubiquitin ligase. This may be because the specific POI cannot act as a substrate or at least not a favorable substrate for the E3-ubiquitin ligase and the degradation process following ubiquitination by the E3-ubiquitin ligase. The main reasons for this may be the expression of E3-ubiquitin ligase and POI in the different cellular compartments, incompatibility of the POI/ E3-ubiquitin ligase to form ternary complex, absence of lysine residue on the POI surface in the vicinity of the E3-ubiquitin ligase machinery, and absence of polyubiquitin chain linkage (like lysine-48 linked ubiquitin chain) activity of the E3-ubiquitin ligase machinery that targets the ubiquitinated POI to 26S proteasome for degradation. Since the effort for synthesizing a PROTAC is relatively high, the inventors of the present invention recognized that there is a general need for a screening system for testing whether a specific protein of interest can act as a substrate, in particular a favorable substrate, for a specific enzyme.

From Qian, Shu-Bing et al., J. Biol. Chem. 284, 2009, pages 26797 to 26802, the use of an engineered version of the carboxyl terminus of Hsc70-interacting protein ubiquitin ligase (CHIP) as a model E3 system to investigate the role of substrate positioning in its ubiquitination is known. One domain of this CHIP was replaced with FK506 binding protein (FKBP) without resulting in a loss of its enzymatic activity. FKBP and FKBP12-rapamycin binding domain (FRB) dimerize with high affinity in the presence of rapamycin. This property was used to test whether the ligase activity of the CHIP-FKBP construct can be redirected toward new substrates. Therefore, a FRB-fused green fluorescence protein (GFP) was constructed. The authors found that addition of rapamycin to the FRB-GFP and the FKBP-CHIP resulted in efficient ubiquitin conjugation on FRB-GFP.

Lazarou, M. et al., Developmental Cell 22, 2012, pages 320 to 333, discloses an assay in which the FKBP domain was fused to mCherry-Parkin, wherein Parkin is a cytosolic E3 ligase. Furthermore, the mitochondrial protein Fis1 was tagged with FRB. Upon treatment of cells containing both constructs with a rapalog, mCherry-FKBP-Parkin was recruited to mitochondria. However, the mCherry-FKBP-Parkin targeted to mitochondria by FRB-Fis1 did not ubiquitinate mitochondria.

From Pinch, B. J. et al., "A Strategy to Assess the Cellular Activity of E3 Ligases against Neo-Substrates using Electrophilic Probes", bioRxiv, 2020 (https://doi.org/10.1101/2020.08.13.249482) a method for evaluating the ability of recombinant E3 ligase components to support neo-substrate degradation is known. The method makes use of Covalent Functionalization Followed by E3 Electroporation (COFFEE) into live cells. For example a recombinant von Hippel-Lindau (VHL) E3 ligase was functionalized via its solvent-exposed cysteines using a simple maleimide warhead linked to the BRD4 ligand JQ1. This functionalized recombinant E3 ligase was then electroporated into live cells to form functional E3-ubiquitin ligase complexes capable of catalyzing degradation of the target protein BRD4. Degradation of BRD4 was assessed by immunoblot analysis.

The purpose of the present invention is to provide an alternative system and method as well as a use of the system for checking whether a specific protein of interest (POI) can act as a substrate for a specific enzyme selected from an E3-ubiquitin ligase, a SUMO E3 ligase, a deubiquitinating enzyme, a kinase or a phosphatase.

The problem of the invention is solved by present claims 1, 9 and 11. Embodiments of the invention are subject-matter of claims 2 to 8, 10 and 12 to 15.

According to the invention a system for checking whether a protein of interest (POI) can act as a substrate, in particular a favorable substrate, for an enzyme selected from an E3-ubiquitin ligase, a SUMO E3 ligase, a deubiquitinating enzyme, a kinase or a phosphatase in a eukaryotic cellular environment is provided. The system comprises
- a first recombinant protein comprising or consisting of
   - an amino acid sequence of the enzyme and an amino acid sequence of a first binding protein of a dimerization system dimerizing the first binding protein with a second binding protein upon addition of a ligand, wherein a first linker is optionally arranged between the amino acid sequence of the enzyme and the amino acid sequence of the first binding protein
   or
- a first recombinant nucleic acid coding for the first recombinant protein,
- a second recombinant protein comprising or consisting of
   - an amino acid sequence of the POI and an amino acid sequence of the second binding protein of the dimerization system, wherein a second linker is optionally arranged between the amino acid sequence of the POI and the amino acid sequence of the second binding protein, and
   - an amino acid sequence of a reporter protein or reporter peptide, wherein a third linker is optionally arranged between the amino acid sequence of the POI and the amino acid sequence of the reporter protein or reporter peptide or between the amino acid sequence of the second binding protein and the amino acid sequence of the reporter protein or reporter peptide,
   or
- a second recombinant nucleic acid coding for the second recombinant protein
   and
- the ligand.

In case the POI can act as a substrate for one of the mentioned enzymes, the POI is ubiquitinated in case the enzyme is an E3-ubiquitin ligase, SUMOylated in case the enzyme is a SUMO E3 ligase, deubiquitinated in case the enzyme is a deubiquitinating enzyme, phosphorylated, in case the enzyme is a kinase or dephosphorylated in case the enzyme in a phosphatase. As a consequence, the POI is degraded, e. g. in case the POI is ubiquitinated, or stabilized, e. g. in case the POI is deubiquitinated, in a eukaryotic cellular environment. In case the POI is SUMOylated the SUMOylation may either stabilize the POI by preventing ubiquitination and proteasomal degradation or destabilized by mediating UPS mediated degradation. In any case the enzymatic activity can change the cellular concentration of the POI. The eukaryotic cellular environment may be provided in a mammalian cell, a fungal cell, in particular a yeast cell, or a plant cell. All eukaryotic cells comprise an E3 machinery and proteasome, i. e. an Ubiquitin Proteasome System (UPS). The eukaryotic cellular environment may be any environment comprising an E3 machinery and proteasome, i. e. the Ubiquitin Proteasome System (UPS).

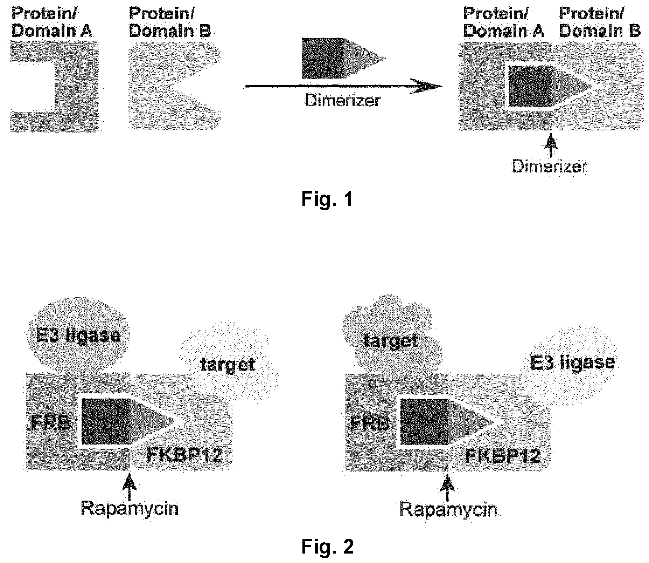

The dimerization system is a so called chemically induced dimerization system. It may comprise or a consist of the first binding protein, the second binding protein and the ligand. The first binding protein and the second binding protein may be identical or different. In case the first binding protein and the second binding protein are different, the dimerization system is a heterodimerization system. In an embodiment of the invention the dimerization system is a heterodimerization system which is schematically shown in Fig. 1, wherein the ligand is designated as "Dimerizer".

Any of the first linker, the second linker and the third linker may independently from each other consist of glycine and/or serine residues or any of the first linker, the second linker and the third linker may independently from each other comprise or consist of any of the sequences SEQ ID NO 3 to SEQ ID NO 8 or an oligomer, in particular a dimer, trimer, tetramer, pentamer, hexamer, heptamer, octamer or nonamer, of any of these sequences, in particular of any of sequences SEQ ID NO 3, SEQ ID NO 4 and SEQ ID NO 5, in particular of sequence SEQ ID NO 3 in particular a dimer of SEQ ID NO 3 which is identical with SEQ ID NO 4.

SEQ ID NO 3 to SEQ ID NO 8 are as follows:
SEQ ID NO 3: GSSG
SEQ ID NO 4: GSSGGSSG
SEQ ID NO 5: GGGS
SEQ ID NO 6: GAATTCGGGAGCTCCGGTGGGAGCTCCGGT
SEQ ID NO 7: ACGCGTGGGAGCTCCGGTGGGAGCTCCGGT
SEQ ID NO 8: GGGAGCTCCGGTGGGAGCTCCGGTACGCGT

The principle of the present invention is that the system comprises components that allow to bring the POI and the enzyme in proximity to each other in a eukaryotic cellular environment by dimerization of the first binding protein part of the first recombinant protein and the second binding protein part of the second recombinant protein by use of the ligand. For this purpose the first recombinant protein and the second recombinant protein can be expressed in a eukaryotic cell and the ligand can be introduced into the cells e. g. by use of electroporation and/or a substance promoting cellular uptake such as dimethyl sulfoxide (DMSO). The principle of the present invention is schematically shown in Fig. 2 except that the reporter protein or reporter peptide at the POI is missing. In Fig. 2 the POI is designated as "target", the enzyme E3-ubiquitin ligase is designated as "E3 ligase", the first binding protein is FRB in the left part of Fig. 2 and FKBP12 in the right part of Fig.2, the second binding protein is FKBP12 in the left part of Fig. 2 and FRB in the right part of Fig.2 and the ligand is rapamycin. As can be seen from a comparison of the left part and the right part of Fig. 2, it is irrelevant whether the amino acid sequence of the first binding protein in the first recombinant protein comprising the enzyme and the amino acid sequence of the second binding protein in the second recombinant protein comprising the POI (target) is that of FRB or that of FKBP12. It is only important that the first binding protein and the second binding protein are chosen such that the first binding protein can bind to the second binding protein via the ligand.

The change in POI concentration can be detected by use of the reporter protein or reporter peptide. The reporter protein or reporter peptide is any protein or peptide that can be directly or indirectly, e. g. after conversion of a substrate, be detected by physical means, in particular by detection of luminescence, in particular fluorescence or chemiluminescence. In this way suitable combinations of POIs and enzymes in which the POIs can act as substrates for the enzymes can be found with very limited effort. It is in particular not required to perform chemical synthesis for finding suitable combinations of POIs and enzymes as it is the case when a Proteolytic Targeting Chimera (PROTAC) is generated for this purpose. The first recombinant protein and the second recombinant protein can be generated by genetic engineering. Furthermore, compared to results obtainable by use of a PROTAC the use of the system according to the invention can provide results in shorter time with less costs. Due to the reporter protein or reporter peptide the readout of the results is simple and enables a use of the system according to the invention in high-throughput screening. The system and its use are well suited for commercial use. The system may be provided as a kit for checking whether a protein of POI can act as a substrate, in particular a favorable substrate, for an enzyme selected from an E3-ubiquitin ligase, a SUMO E3 ligase, a deubiquitinating enzyme, a kinase or a phosphatase in a eukaryotic cellular environment, wherein the kit comprises all components of the system according to the invention. However, it is also possible to provide the system according to the invention by separately procuring and then combining the individual components of the system. It is also possible to provide the system according to the invention by providing a kit comprising a part of the components of the system and to procure the part(s) of the system not comprised by the kit separately and then combining the kit with the part(s) of the system not comprised by the kit.

In case the enzyme is E3-ubiquitin ligase, the synthesis of a successful PROTAC is very promising for the combinations of POI and enzyme for which it has been found by use of the system according to the invention that the POI can act as a substrate for the enzyme. Since the effort for synthesizing a PROTAC is relatively high and many PROTACs do not work because the POI cannot act as a substrate for a specific enzyme the system according to the invention reduces the effort for finding successful PROTACs.

The first binding protein of the dimerization system may be FKBP-rapamycin binding domain of mTOR complex 1 (FRB) or its analog FRB^{T78L} or another analog of FRB and the second binding protein of the dimerization system may be 12-kDa FK506 binding protein (FKBP12) or an analog thereof.

Alternatively, the first binding protein of the dimerization system may be 12-kDa FK506 binding protein (FKBP12) or an analog thereof and the second binding protein of the dimerization system may be FKBP-rapamycin binding domain of mTOR complex 1 (FRB) or its analog FRB^{T78L} or another analog of FRB.

In both of the above cases the ligand may be rapamycin or an analog of rapamycin which analog is able to dimerize FRB or FRB^{T78L} and FKBP12. In particular the analog may be AP21967 in case that the first binding protein or the second binding protein is FRB^{T78L}. Alternatively, the analog may be temsirolimus, everolimus, ridaforolimus, umirolimus, or zotarolimus. However, in principle any chemically induced dimerization system can be used.

Any of the before mentioned analogs of FRB and FKBP12 consists of an amino acid sequence having a sequence identity of at least 93 % or a similarity of at least 90 % to the amino acid sequence of FRB and FKBP12, respectively. The amino acid sequence of FRB may be amino acid sequence SEQ ID NO 11. The amino acid sequence of FKBP12 may be amino acid sequence SEQ ID NO 13. The sequence similarity is calculated according to the EMBOSS needle algorithm having an open gap penalty of 10.0 and an extended gap penalty of 0.5 and using the Blosum 62 matrix.

Sequence similarity in the context of the present invention is determined according to the EMBOSS needle algorithm. The EMBOSS needle algorithm is the standard algorithm for aligning a first amino acid sequence, in particular a first protein, a first polypeptide residue or a first oligopeptide residue, and a second amino acid sequence, in particular a second protein, a second polypeptide residue or a second oligopeptide residue, over the whole length of both the first amino acid sequence and the second amino acid sequence. The EMBOSS needle algorithm implements the Needleman-Wunsch algorithm (Needleman S.B. and Wunsch C.D., 1970 J. Mol. Biol. 48, 443 - 453), wherein a penalty for a gap of n positions is computed according to the following formula: open gap penalty + n - 1 x extended gap penalty.

The entire length of the first amino acid sequence and the second amino acid sequence is aligned, and there is no penalty for hanging ends of the overlap.

A "gap" designates one or more amino acid residue(s) not being identical in the aligned sequences. The open gap penalty in the context of the present invention is 10.0. The extended gap penalty in the context of the present invention is 0.5. The scoring matrix for comparing amino acid similarities in the context of the present invention is the Blosum 62 matrix. The open gap penalty, the extended gap penalty and the Blosum 62 matrix are standard parameters used in the art. In pairwise sequence alignment, the open gap penalty refers to the penalty for opening a gap in the alignment. The open gap penalty does not penalize terminal gaps. In pairwise sequence alignment, the extended gap penalty refers to the penalty for extending a gap by one residue. The extended gap penalty does not penalize terminal gaps. Sequence alignments can be performed with these parameters, e.g. by use of publicly available free tools. For example, EMBL-EBI offers in its assortment of internet tools, e. g. under https://www.ebi.ac.uk/Tools/psa/emboss_needle/, a free pairwise sequence alignment service in which the parameters of the present invention can be chosen.

Examples of suitable dimerization systems are as follows:
1) GAI (gibberellin insensitive) and GID1 (gibberellin insensitive dwarf 1) system:
   a) GAI-tag sequence (91 amino acids): SEQ ID NO 16
   b) GID1-tag sequence (344 amino acids): SEQ ID NO 17
   c) Dimerization agent:
      (1) Gibberellic acid:
         IUPAC:
         (1S,2S,4aR,4bR,7S,9aS,10S,10aR)-2,7-dihydroxy-1-methyl-8-methylene-13-oxo-1,2,4b,5,6,7,8,9,10,10a-decahydro-4a,1-(epoxymethano)-7,9a-methanobenzo[a]azulene-10-carboxylic acid
         or
      (2) Gibberellic acid acetoxymethyl ester:
         IUPAC:
         acetoxymethyl (1S,2S,4aR,4bR,7S,9aS,10S,10aR)-2,7-dihydroxy-1-methyl-8-methylene-13-oxo-1,2,4b,5,6,7,8,9,10,10a-decahydro-4a,1-(epoxymethano)-7,9a-methanobenzo[a]azulene-10-carboxylate
2) Halo-tag (engineered Haloalkane dehalogenase) and SNAP-Tag (Mutants of the DNA repair protein O6-alkylguanine-DNAalkyltransferase)
   a) Halo-tag sequence (296 amino acids): SEQ ID NO 18
   b) SNAP-Tag sequence (181 amino acids): SEQ ID NO 19
   c) Dimerization agent:
      HAXS8:
      IUPAC:
      N-[[4-[[(2-Amino-9-H-purin-6-yl)oxy]methyl]phenyl]methyl]-2-[4-[(18-chloro-3,6,9,12-tetraoxaoctadec-1yl)oxy]-2,3,5,6-tetrafluorophenoxy]acetamide
3) ABI (Abscisic Acid insensitive) and PYL1 (Pyrabactin resistance like)
   a) ABI-tag sequence (298 amino acids): SEQ ID NO 20
   b) PYL1-tag sequence (176 amino acids): SEQ ID NO 21
   c) Dimerizing agent:
      Abscisic acid:
      IUPAC:
      (2Z,4E)-5-[(1S)-1-hydroxy-2,6,6-trimethyl-4-oxocyclohex-2-en-1-yl]-3-methylpenta-2,4-dienoic acid
4) ABI (Abscisic Acid insensitive) and PYR1 (Pyrabactin resistance 1)
   a) ABI-tag sequence (298 amino acids): SEQ ID NO 20
   b) PYR1-tag sequence (190 amino acids): SEQ ID NO 22
   c) Dimerizing agent:
      Mandipropamid:
      IUPAC:
      2-(4-chlorophenyl)-N-[2-(3-methoxy-4-prop-2-ynoxyphenyl)ethyl]-2-prop-2-ynoxyacetam ide
5) DHFR (*E*. *coli* dihydrofolate reductase) and Halo-tag (engineered Haloalkane dehalogenase)
   a) DHFR-tag sequence (158 amino acids): SEQ ID NO 23
   b) Halo-tag sequence (296 amino acids): SEQ ID NO 18
   c) Dimerizing agent:
      Trimethoprim-HaloTag ligand (TMP-Htag):
      IUPAC:
      N1-(2-(2-((6-chlorohexyl)oxy)ethoxy)ethyl)-N4-(3-(4-((2,4-diaminopyrimidin-5-yl)methyl)-2,6-dimethoxyphenoxy)propyl)succinamide

The sequence identity may be at least 95 %, in particular at least 96 %, in particular at least 97 %, in particular at least 98 %, in particular at least 99 % and at most less than 100 % , in particular at most 99.99 %. Sequence similarity may be at least 95 %, in particular at least 96 %, in particular at least 97 %, in particular at least 98 %, in particular at least 99 % and at most less than 100 % , in particular at most 99.99 %.

In case the enzyme acts on lysine residue(s) of the POI it may be that it also acts on lysine residue(s) of the second binding protein and/or lysine residues of the reporter protein or reporter peptide and/or of the second linker and/or of the third linker in case the second linker and/or the third linker is/are present in the second recombinant protein. For example, this may be the case when the enzyme is E3-ubiquitin ligase, the reporter protein is NanoLuc^{®} and the second binding protein is FKBP12. In case of ubiquitination of FKBP12 and/or NanoLuc^{®} followed by degradation of the second recombinant protein it would not be possible to determine whether degradation was caused by ubiquitination of the POI, of FKBP12 or of NanoLuc^{®}. For restricting the potential activity of the enzyme to the POI, the second recombinant protein may be constructed such that in the second recombinant protein
- the reporter protein or reporter peptide and the second binding protein and the second linker and/or third linker in case of presence in the second recombinant protein are chosen such that no lysine residue is present in the amino acid sequences of the reporter protein or reporter peptide, the second binding protein and the second linker and/or third linker
   or
- any lysine residue in the amino acid sequence of the reporter protein or reporter peptide, in the amino acid sequence of the second binding protein, in particular of FKBP12 or the analog thereof or of FRB or of its analog FRB^{T78L} or of the other analog of FRB, and in the amino acid sequence of the second linker and/or third linker in case of presence in the second recombinant protein is replaced by an arginine residue or histidine residue, in particular an arginine residue.

In an embodiment of the invention the system comprises the first recombinant protein and the second recombinant protein and the ligand. In another embodiment of the invention the system comprises the first recombinant nucleic acid and the second recombinant nucleic acid and the ligand. The first recombinant nucleic acid may be contained in an expression vector, in particular a plasmid or a virus or a lentiviral vector. The second recombinant nucleic acid may be contained in the same or a further expression vector, in particular the plasmid or a further plasmid or the virus or a further virus or the lentiviral vector or a further lentiviral vector. The expression vector and the further expression vector may be in each case independently from each other a plasmid or a virus. Alternatively, the first and the second recombinant nucleic acid may be contained in the genome of an animal cell, in particular a mammalian cell, after introduction by CRISPR knock-in technique. In particular the amino acid sequence of an E3-ubiquitin ligase can be coupled, i. e. tagged, with the amino acid sequences of the first binding protein and/or the amino acid sequence of an oncogenic protein, such as MYC, WDR5 and AURKA, can be coupled, i. e. tagged, with the amino acid sequences of the second binding protein and of the reporter protein or reporter peptide in their endogenous genomic locus using CRISPR knock-in. In case of envisaged tumor treatment, the E3-ubiquitin ligase can be an E3-ubiquitin ligase that is tissue specific and tissue essential.

The reporter protein or reporter peptide may be a luciferase, in particular firefly-luciferase, 19 kDa catalytic subunit of Oplophorus luciferase, in particular a luciferase having sequence SEQ ID NO 1 (NanoLuc^{®}), or a luciferase having sequence SEQ ID NO 2 (HiBiT), or a fluorophore, in particular green fluorescent protein (GFP), red fluorescent protein (RFP), cyan fluorescent protein(CFP), blue fluorescent protein (BFP), yellow fluorescent protein (YFP), orange fluorescent protein (OFP), far-red fluorescent protein, infra-red fluorescent protein, mNeonGFP (mNG), enhanced GFP (EGFP) or mCherry. EGFP is a basic green fluorescent protein derived from Aequorea Victoria.

The E3-ubiquitin ligase may be von Hippel-Lindau E3-ubiquitin ligase (VHL) or Cereblon E3-ubiquitin ligase (CRBN). The POI may be an oncoprotein, i. e., a protein promoting cancer growth or having a gain of function mutation in a cancer cell. The oncoprotein may be oncoprotein Myc, oncoprotein MycN , oncoprotein MycL, aurora kinase A (AURKA), aurora kinase B (AURKB), BCR-ABL fusion protein, signal transducer and activator of transcription 3 (STAT3), GTPase Kras (KRAS), EWS-FLI fusion protein, epidermal growth factor receptor (EGFR), Erb-b2 receptor tyrosine kinase 2 (ERBB2), ALK tyrosine kinase receptor (ALK), Wee1-like protein kinase (WEE1), catenin beta-1 (CTNNB1) or Yes-associated protein 1 (YAP1).

The POI may also be a cofactor of an oncoprotein, i. e., a protein promoting the oncogenicity of an oncoprotein. The cofactor may be WD repeat-containing protein 5 (WDR5), RuvB-like AAA ATPase 1 (RUVBL1), RuvB-like AAA ATPase 2 (RUVBL2), transcription elongation factor SPT5 (SUPT5H), transcription elongation factor SPT6 (SUPT6H), transcription elongation factor SPT4 (SUPT4H), transformation/transcription domain-associated protein (TRRAP) or HECT-UBA and WWE domain containing E3 ubiquitin protein ligase 1 (HUWE1).

The POI may also be a protein responsible for immune system deregulation and induced autoimmunity such as Bruton tyrosine kinase (BTK), interleukin 1 receptor associated kinase 4 (IRAK4), interleukin 1 receptor associated kinase 3 (IRAK3) or mitogen-activated protein kinase kinase kinase kinase 1 (MAP4K1).

The POI may also be a neurotoxic protein or protein aggregate that promote a neurodegenerative disease such as microtubule associated protein tau (MAPT), mutant huntingtin (mHtt), TAR RNA-binding protein (TARDBP), FIS RNA-binding protein (FUS), glycogen synthase kinase 3 beta (GSK3B), or synuclein alpha (SNCA).

The invention further concerns the use of the system according to the invention for checking whether a POI can act as a substrate, in particular a favorable substrate, for an enzyme selected from an E3-ubiquitin ligase, a SUMO E3 ligase, a deubiquitinating enzyme, a kinase or a phosphatase, wherein the first recombinant protein, the second recombinant protein and the ligand are brought into contact in an eukaryotic cellular environment, i. e. in eukaryotic cells, e. g. by electroporation, or an environment comprising all constituents of an eukaryotic cellular environment. An environment comprising all constituents of an eukaryotic cellular environment can be provided in the form of a lysate of eukaryotic cells. It is also possible that the first recombinant nucleic acid and the second recombinant nucleic acid are co-expressed in eukaryotic cells in cell culture followed by a treatment of the cells with the ligand. In both cases the change of cellular concentration of the POI may be monitored by measuring the amount or concentration of the reporter protein or reporter peptide in the eukaryotic cells, in the environment comprising all constituents of an eukaryotic cellular environment or in medium in which the eukaryotic cells are suspended. The treatment of the cells with the ligand may occur by addition of the ligand to the cells together with a substance promoting cellular uptake such as dimethyl sulfoxide (DMSO).

The reporter protein or reporter peptide may be a luciferase, wherein its amount or concentration in the cells or in the medium is measured by measuring of luminescence after conversion of a further substrate by the luciferase. The further substrate may be introduced into the cells or added to the environment comprising all constituents of an eukaryotic cellular environment. It is also possible that the cells are lysed and subsequently mixed with the further substrate. The lysing of the cells and mixing with the further substrate may be performed in the medium.

The invention further concerns a method for checking whether a POI can act as a substrate, in particular a favorable substrate, for an enzyme selected from an E3-ubiquitin ligase, a SUMO E3 ligase, a deubiquitinating enzyme, a kinase or a phosphatase by use of the system according to the invention. The method may comprise the following steps:
- amplifying nucleic acid fragments coding for the enzyme, for a first binding protein of a dimerization system dimerizing the first binding protein with a second binding protein upon addition of a ligand, for the second binding protein of the dimerization system, for the reporter protein or reporter peptide, for the POI and optionally for the first, the second and/or the third linker by use of polymerase chain reaction (PCR), wherein the reporter protein or reporter peptide is an enzyme, in particular a luciferase,
- cloning the amplified nucleic acid fragments into one or more expression vectors such that the resulting nucleic acid or nucleic acids code for the first recombinant protein and the second recombinant protein,
- transformation of competent bacteria with the vector(s), selecting and expanding bacteria containing the vector(s) and extracting the amplified vector(s),
- culturing eukaryotic cells, in particular mammalian, fungal or plant cells, in cell culture,
- transfection of the vector(s) into the eukaryotic cells,
- expressing the first recombinant protein and the second recombinant protein in the eukaryotic cells,
- treating the eukaryotic cells with the ligand, optionally in combination with a cell membrane penetration promoting substance,
- lysing the eukaryotic cells, adding a further substrate for the reporter protein or reporter peptide and (an) additional substance(s) in case the additional substance(s) is/are required for a conversion of the further substrate by the reporter protein or reporter peptide and determining whether a conversion of the further substrate changes over time, wherein a change of the conversion is an indication for the acting of the POI as a substrate for the enzyme.

The eukaryotic cells may be mammalian cells. The mammalian cells may be HEK-293 cells. HEK-293 cells are cells of a human embryonic kidney cell line. The first binding protein of the dimerization system may be FKBP-rapamycin binding domain of mTOR complex 1 (FRB) or its analog FRB^{T78L} or another analog of FRB, the second binding protein of the dimerization system may be 12-kDa FK506 binding protein (FKBP12) or an analog thereof and the ligand may be rapamycin or an analog of rapamycin which analog is able to dimerize FRB or FRB^{T78L} and FKBP12. Analogs of rapamycin are generally referred to as "rapalogs". The rapalog may be AP21967 in case that the first binding protein is FRB^{T78L}, temsirolimus, everolimus, ridaforolimus, umirolimus, or zotarolimus. Any of the before mentioned analogs of FRB and FKBP12 consists of an amino acid sequence having a sequence identity of at least 93 % or a similarity of at least 90 % to the amino acid sequence of FRB and FKBP12, respectively, wherein the sequence similarity is calculated according to the EMBOSS needle algorithm having an open gap penalty of 10.0, an extended gap penalty of 0.5 and using the Blosum 62 matrix.

The rapalogs (rapamycin analogues) that can be used and their IUPAC formula are as follows:
1) Temsirolimus (CCI-779):
   IUPAC:
   [(1*R*,2*R*,4*S*)-4-[(2*R*)-2-[(1*R*,9*S*,12*S*,15*R*,16*E*,18*R*,19*R*,21*R*,23*S*,24*E*,26*E*,28*E*,30*S*,32*S*,35*R*)-1,18-dihydroxy-19,30-dimethoxy-15,17,21,23,29,35-hexamethyl-2,3,10,14,20-pentaoxo-11,36-dioxa-4-azatricyclo[30.3.1.0^{4,9}]hexatriaconta-16,24,26,28-tetraen-12-yl]propyl]-2-methoxycyclohexyl] 3-hydroxy-2-(hydroxymethyl)-2-methylpropanoate
2) Everolimus (RAD001): 42-O-(2-hydroxyethyl)rapamycin
   IUPAC:
   (1*R*,9*S*,12*S*,15*R*,16*E*,18*R*,19*R*,21*R*,23*S*,24*E*,26*E*,28*E*,30*S*,32*S*,35*R*)-1,18-dihydroxy-12-[(2*R*)-1-[(1*S*,3*R*,4*R*)-4-(2-hydroxyethoxy)-3-methoxycyclohexyl]propan-2-yl]-19,30-dimethoxy-15,17,21,23,29,35-hexamethyl-11,36-dioxa-4-azatricyclo[30.3.1.0^{4,9}]hexatriaconta-16,24,26,28-tetraene-2,3,10,14,20-pentone
3) Ridaforolimus (Deforolimus, MK-8669, AP23573):
   IUPAC:
   (1*R*,9*S*,12*S*,15*R*,16*E*,18*R*,19*R*,21*R*,23*S*,24*E*,26*E*,28*E*,30*S*,32*S*,35*R*)-12-[(2*R*)-1-[(1*S*,3*R*,4*R*)-4-dimethylphosphoryloxy-3-methoxycyclohexyl]propan-2-yl]-1,18-dihydroxy-19,30-dimethoxy-15,17,21,23,29,35-hexamethyl-11,36-dioxa-4-azatricyclo[30.3.1.0^{4,9}]hexatriaconta-16,24,26,28-tetraene-2,3,10,14,20-pentone
4) Umirolimus (Biolimus A9)
   IUPAC:
   (1*R*,9*S*,12*S*,15*R*,16*E*,18*R*,19*R*,21*R*,23*S*,24*E*,26*E*,28*E*,30*S*,32*S*,35*R*)-12-[(2*R*)-1-[(1*S*,3*R*,4*R*)-4-(2-ethoxyethoxy)-3-methoxycyclohexyl]propan-2-yl]-1,18-dihydroxy-19,30-dimethoxy-15,17,21,23,29,35-hexamethyl-11,36-dioxa-4-azatricyclo[30.3.1.0^{4,9}]hexatriaconta-16,24,26,28-tetraene-2,3,10,14,20-pentone
5) Zotarolimus (ABT-578):
   IUPAC:
   (1*R*,9*S*,12*S*,15*R*,16*E*,18*R*,19*R*,21*R*,23*S*,24*E*,26*E*,28*E*,30*S*,32*S*,35*R*)-1,18-dihydroxy-19,30-dimethoxy-12-[(2*R*)-1-[(1*S*,3*R*,4*S*)-3-methoxy-4-(tetrazol-1-yl)cyclohexyl]propan-2-yl]-15,17,21,23,29,35-hexamethyl-11,36-dioxa-4-azatricyclo[30.3.1.0^{4,9}]hexatriaconta-16,24,26,28-tetraene-2,3,10,14,20-pentone
   Similarly, another rapalog, AP21967 can also be used. But it requires mutated version of FRB (FRB^{T78L}).
6) AP21967:
   IUPAC:
   (1R,9S,12S,14S,15R,16E,18R,19S,20S,21R,23S,24E,26E,28E,30S,32S,35R)-1,14,18,20-tetrahydroxy-12-[(2R)-1-[(1S,3R,4R)-4-hydroxy-3-methoxycyclohexyl]propan-2-yl]-19-methoxy-15,17,21,23,29,35-hexamethyl-30-(7-methyl-1H-indol-3-yl)-11,36-dioxa-4-azatricyclo[30.3.1.04,9]hexatriaconta-16,24,26,28-tetraene-2,3,10-trione

The E3-ubiquitin ligase may be von Hibbel-Lindau E3-ubiquitin ligase (VHL) or Cereblon E3-ubiquitin ligase (CRBN). The POI may be oncoprotein Myc, oncoprotein MycN , oncoprotein MycL, aurora kinase A (AURKA), aurora kinase B (AURKB), BCR-ABL fusion protein, signal transducer and activator of transcription 3 (STAT3), GTPase Kras (KRAS), EWS-FLI fusion protein, epidermal growth factor receptor (EGFR), Erb-b2 receptor tyrosine kinase 2 (ERBB2), ALK tyrosine kinase receptor (ALK), Wee1-like protein kinase (WEE1), catenin beta-1 (CTNNB1), Yes-associated protein 1 (YAP1), WD repeat-containing protein 5 (WDR5), RuvB-like AAA ATPase 1 (RUVBL1), RuvB-like AAA ATPase 2 (RUVBL2), transcription elongation factor SPT5 (SUPT5H), transcription elongation factor SPT6 (SUPT6H), transcription elongation factor SPT4 (SUPT4H), transformation/transcription domain-associated protein (TRRAP), HECT-UBA and WWE domain containing E3 ubiquitin protein ligase 1 (HUWE1), Bruton tyrosine kinase (BTK), interleukin 1 receptor associated kinase 4 (IRAK4), interleukin 1 receptor associated kinase 3 (IRAK3), mitogen-activated protein kinase kinase kinase kinase 1 (MAP4K1), microtubule associated protein tau (MAPT), mutant huntingtin (mHtt), TAR RNA-binding protein (TARDBP), FIS RNA-binding protein (FUS), glycogen synthase kinase 3 beta (GSK3B), or synuclein alpha (SNCA).

The reporter protein or reporter peptide may be a luciferase, in particular firefly-luciferase, 19 kDa catalytic subunit of Oplophorus luciferase, in particular a luciferase having sequence SEQ ID NO 1 (NanoLuc^{®}), or a luciferase having sequence SEQ ID NO2 (HiBiT), or a fluorophore, in particular green fluorescent protein (GFP), red fluorescent protein (RFP), cyan fluorescent protein(CFP), blue fluorescent protein (BFP), yellow fluorescent protein (YFP), orange fluorescent protein (OFP), far-red fluorescent protein, infra-red fluorescent protein, mNeonGFP (mNG), enhanced GFP (EGFP) or mCherry.

The cell membrane penetration promoting substance may be dimethyl sulfoxide (DMSO).

All features indicated in the specification are to be understood as features applicable to all embodiments of the invention. This means, for example, that a feature indicated for the system for checking whether a POI can act as a substrate for an enzyme in a eukaryotic cellular environment can also be applied to the use of the system and the method according to the invention and vice versa.

The invention will be explained in more details in the following embodiments.
- Fig. 1: shows a schematic representation of a chemically induced dimerization/chemically induced proximity of two different proteins or domains.
- Fig. 2: shows the schematic representation of the rapamycin induced proximity between E3-ubiquitin ligase and a POI as a target.
- Fig. 3: shows a vector map of a construct for transient expression of VHL-FRB.
- Fig. 4: shows a vector map of a construct for transient expression of CRBN-FRB.
- Fig. 5: shows a vector map of a construct for transient expression of FRB.
- Fig. 6: shows a vector map of a construct for transient expression of WDR5-NanoLuc^{®}-FKBP12.
- Fig. 7: shows an immunoblot of HEK293 cells transiently transfected with SFFV promoter containing WDR5-FKBP12-luciferase and VHL-FRB or FRB constructs in the ratio of 1:1 or 1:10 (FKBP12: FRB) which cells were treated with 10 nM rapamycin in a solution or the solution without rapamycin for 6 hours, wherein vinculin is used as a control for checking whether each lane of the gel has been uniformly loaded.
- Fig. 8: shows WDR5 levels/concentrations based on luciferase measurement of HEK293 cells transiently expressing WDR5-FKBP12-luciferase and VHL-FRB, CRBN-FRB or FRB at various time points after rapamycin treatment.
- Fig. 9: shows an immunoblot of HEK-293 cells transiently expressing WDR5-FKBP12-luciferase and VHL-FRB or CRBN-FRB or FRB treated with rapamycin in a solution or the solution without rapamycin for 6 hours, wherein vinculin is used as a control for checking whether each lane of the gel has been uniformly loaded.
- Fig. 10: shows WDR5 concentrations/levels based on luciferase measurement of HEK-293 cells transiently expressing WDR5-FKBP12-luciferase, wherein all lysine residues in the FKBP12 and in the luciferase have been replaced by arginine, and VHL-FRB or CRBN-FRB or FRB at various time points after rapamycin treatment.
- Fig. 11: shows an immunoblot of HEK-293 cells transiently expressing WDR5-FKBP12-luciferase, wherein all lysine residues in FKBP12 and in the luciferase have been replaced by arginine residues, and VHL-FRB, CRBN-FRB or FRB treated with rapamycin in a solution or the solution without rapamycin for 6 hours, wherein vinculin is used as a control for the uniformly loading of each lane of the gel.
- Fig. 12: shows an estimated time line for the performance of the method according to the invention.

### Embodiments:

In the embodiments and in the figures "E3 ligase" and "E3-ligase" always means "E3-ubiquitin ligase" and "target" means the POI.

### 1. Exemplary experimental steps for CRISPR knock-in:

1) Design of knock-in tag and generation of the homology directed repair (HDR) template plasmid. Some exemplary designs of HDR templates are as follows:
   a) N-terminal target tagging with FKBP12 and NanoLuc^{®}:
      LHA - Start codon - Selection Marker - P2A - FKBP12 - linker - NanoLuc^{®} - linker - RHA,
      wherein LHA is a left homology arm which consists of the genomic sequence before the start codon of the target protein, RHA is a right homology arm which consists of the genomic sequence after the start codon of the target protein, and P2A is a 2A self-cleaving peptide.
   b) C-terminal target tagging with FKBP12 and NanoLuc^{®}:
      LHA - linker- NanoLuc^{®} - linker- FKBP12 - P2A- Selection Marker - Stop codon - RHA,
      wherein LHA is a left homology arm which consists of the genomic sequence before the stop codon of the target protein, RHA is a right homology arm which consists of the genomic sequence after the stop codon of the target protein, and P2A is 2A self-cleaving peptide.
      In the above example for FKBP12 knock-in, the position of NanoLuc^{®} and FKBP12 can also be interchanged.
   c) N-terminal E3-ligase tagging with FRB:
      LHA - Start codon - Selection Marker - P2A - FRB - linker - RHA,
      wherein LHA is a left homology arm which consists of the genomic sequence before the start codon of the E3-ligase, RHA is a right homology arm which consists of genomic sequence after the start codon of the E3-ligase, and P2A is 2A self-cleaving peptide.
   d) C-terminal E3-ligase tagging with FRB:
      LHA - linker - FRB - P2A - Selection Marker - Stop codon - RHA,
      wherein LHA is a left homology arm which consists of the genomic sequence before the stop codon of the E3-ligase, RHA is a right homology arm which consists of genomic sequence after the stop codon of the E3-ligase, and P2A is 2A self-cleaving peptide.
2) Design of the sgRNA and sgRNA cloning in any Cas9-sgRNA vector, such as PX458 sold by the company Addgene as Plasmid #48138
   sgRNA should ideally be designed for the specific terminus namely around the start codon for N-terminal tagging or around the stop codon for C-terminal tagging. The sgRNA sequence must be followed by the PAM site i.e., NGG for sense strand or CCN for the antisense strand.
3) Transfect the cells with both sgRNA and HDR template plasmids.
4) Post 72 hours of transfection, select the cells using a selection marker introduced into the genomic locus using HDR template.
5) Pick the single colony of the selected cells either by serial dilution, FACS sorting or cloning ring. Expand the colony of singe cell.
6) Identify the positive clones with homozygous knock-in using genotypic PCR or western blot (WB). For genotypic PCR, extract the genomic DNA from the clones and perform PCR using screening primers that bind outside the homology arms. Confirm the correct integration by Sanger sequencing of the PCR product spanning the genomic integration site. For WB, probe the whole membrane with the respective antibodies for target protein or E3-ligase. Successful knock-in should result in increase of the size of PCR product or molecular weight of protein as compared to the PCR product or protein molecular weight from the naive cells.

### 2. Generation of VHL-FRB, CRBN-FRB, FRB and WDR5-NanoLuc^{®}-FKBP12 constructs

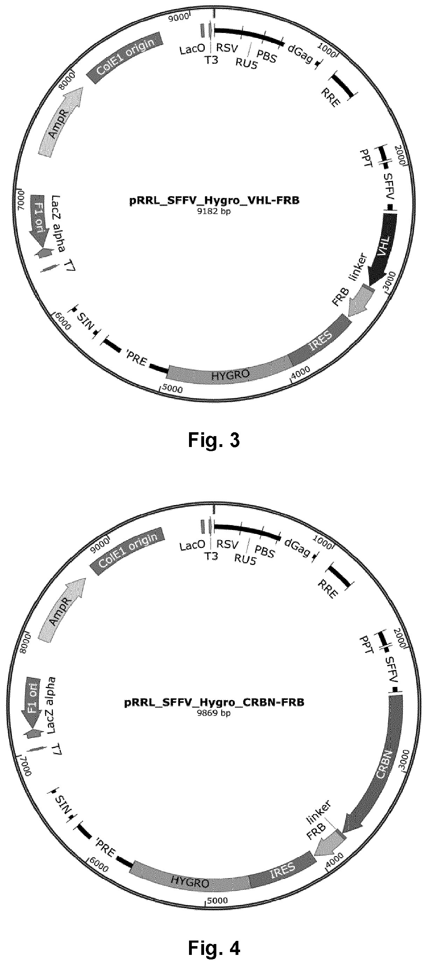

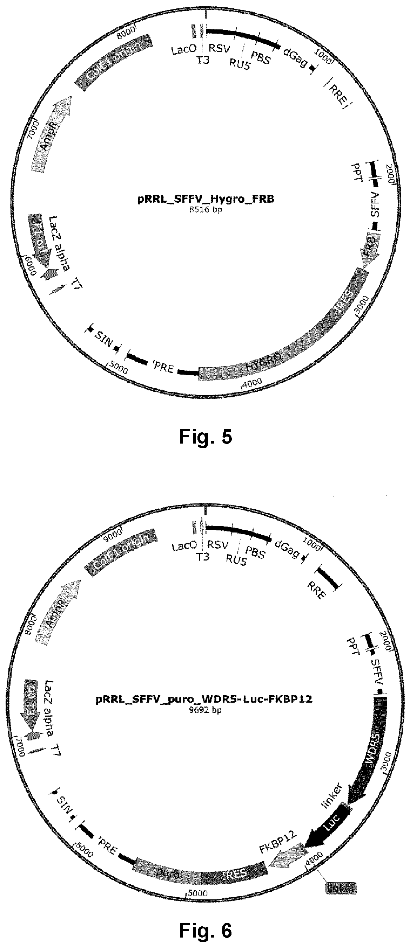

i. Primer pairs were designed with appropriate restriction sites, linkers, start and stop codon. The primers were ordered from Merck KGaA, Deutschland, Sigma-Aldrich to amplify VHL, CRBN, FRB, WDR5, NanoLuc^{®} luciferase and FKBP12 segments of the constructs.
ii. PCR amplification of different segments were carried out with Phusion^{™} polymerase using following templates:
   a. VHL: complementary DNA (cDNA)
   b. CRBN: plasmid from addgene
   c. FRB: complementary DNA (cDNA)
   d. WDR5: plasmid from Promega
   e. NanoLuc^{®}: custom double-stranded DNA fragments (gBlock, ordered from IDT integrated DNA technologies)
   f. FKBP12: plasmid from addgene
iii. Agarose gel electrophoresis of the PCR products were carried out to check the size of the products.
iv. The PCR products with expected band size were gel purified. For this the PCR fragment was cut-out from the agarose gel and extracted using the GeneJET Gel Extraction Kit from the company Thermo Fisher Scientific according to the manufacturer's instructions.
v. Any eukaryotic expression vector can be used for the preparation of the FKBP12 and FRB constructs. In this example, FRB constructs were cloned into pRRLSin.cPPT.WPRE vector with SFFV (Spleen Focus Forming Virus) promoter and Hygromycin as resistance marker. Likewise, FKBP12 construct was cloned into pRRLSin.cPPT.WPRE vector with SFFV promoter and Puromycin as resistance marker.
vi. The PCR amplified fragments and vector backbone (pRRLSin.cPPT:WPRE) were digested with appropriate restriction enzymes.
vii. The digested PCR fragments and vector backbone were gel purified.
viii. The concentration of the digested products was measured using Multiscan Ascent Spectrofluorometer NanoDrop 1000 from Thermo Fisher Scientific.
ix. The digested PCR fragments (inserts) and vector backbone were ligated in a molar ratio of 3:1 (insert : vector backbone).
x. The ligated constructs were transformed into chemically competent *E*. *coli* XL1 blue and bacteria were streaked onto LB-agar plates with ampicillin (pRRL vector consists of ampicillin resistance).
xi. Single colonies of transformed bacteria were propagated in LB medium with ampicillin. Plasmid was isolated and purified from the bacterial culture using PureLink HiPure Plasmid Maxiprep Kit according to the manufacturer's protocol.
xii. The sequence of the plasmid was confirmed by Sanger sequencing.
xiii. The sequence of the fragments of different constructs are as follows:
   a. VHL-FRB
      VHL-linker-FRB: SEQ ID NO 9 
ATGCCCCGGAGGGCGGAGAACTGGGACGAGGCCGAGGTAGGC
GCGGAGGAGGCAGGCGTCGAAGAGTACGGCCCTGAAGAAGAC
GGCGGGGAGGAGTCGGGCGCCGAGGAGTCCGGCCCGGAAGA
GTCCGGCCCGGAGGAACTGGGCGCCGAGGAGGAGATGGAGG
CCGGGCGGCCGCGGCCCGTGCTGCGCTCGGTGAACTCGCGC
 
GAGCCCTCCCAGGTCATCTTCTGCAATCGCAGTCCGCGCGTCG
TGCTGCCCGTATGGCTCAACTTCGACGGCGAGCCGCAGCCCTA
CCCAACGCTGCCGCCTGGCACGGGCCGCCGCATCCACAGCTA
CCGAGGTCACCTTTGGCTCTTCAGAGATGCAGGGACACACGAT
GGGCTTCTGGTTAACCAAACTGAATTATTTGTGCCATCTCTCAAT
GTTGACGGACAGCCTATTTTTGCCAATATCACACTGCCAGTGTA
TACTCTGAAAGAGCGATGCCTCCAGGTTGTCCGGAGCCTAGTC
AAGCCTGAGAATTACAGGAGACTGGACATCGTCAGGTCGCTCT
ACGAAGATCTGGAAGACCACCCAAATGTGCAGAAAGACCTGGA
GCGGCTGACACAGGAGCGCATTGCACATCAACGGATGGGAGAT
GAATTCGGGAGCTCCGGTGGGAGCTCCGGTATCCTCTGGCATG
AGATGTGGCATGAAGGCCTGGAAGAGGCATCTCGTTTGTACTTT
GGGGAAAGGAACGTGAAAGGCATGTTTGAGGTGCTGGAGCCCT
TGCATGCTATGATGGAACGGGGCCCCCAGACTCTGAAGGAAAC
ATCCTTTAATCAGGCCTATGGTCGAGATTTAATGGAGGCCCAAG
AGTGGTGCAGGAAGTACATGAAATCAGGGAATGTCAAGGACCT
CACCCAAGCCTGGGACCTCTATTATCATGTGTTCCGACGAATCT
CAAAGTGA
   b. CRBN-FRB
      CRBN-linker-FRB: SEQ ID NO 10 
ATGGCCGGCGAAGGAGATCAGCAGGACGCTGCGCACAACATG
GGCAACCACCTGCCGCTCCTGCCTGCAGAGAGTGAGGAAGAA
GATGAAATGGAAGTTGAAGACCAGGATAGTAAAGAAGCCAAAAA
ACCAAACATCATAAATTTTGACACCAGTCTGCCGACATCACATA
CATACCTAGGTGCTGATATGGAAGAATTTCATGGCAGGACTTTG
CACGATGACGACAGCTGTCAGGTGATTCCAGTTCTTCCACAAGT
GATGATGATCCTGATTCCCGGACAGACATTACCTCTTCAGCTTT
TTCACCCTCAAGAAGTCAGTATGGTGCGGAATTTAATTCAGAAA
GATAGAACCTTTGCTGTTCTTGCATACAGCAATGTACAGGAAAG
GGAAGCACAGTTTGGAACAACAGCAGAGATATATGCCTATCGA
 
GAAGAACAGGATTTTGGAATTGAGATAGTGAAAGTGAAAGCAAT
TGGAAGACAAAGGTTCAAAGTCCTTGAGCTAAGAACACAGTCAG
ATGGAATCCAGCAAGCTAAAGTGCAAATTCTTCCCGAATGTGTG
TTGCCTTCAACCATGTCTGCAGTTCAATTAGAATCCCTCAATAAG
TGCCAGATATTTCCTTCAAAACCTGTCTCAAGAGAAGACCAATG
TTCATATAAATGGTGGCAGAAATACCAGAAGAGAAAGTTTCATT
GTGCAAATCTAACTTCATGGCCTCGCTGGCTGTATTCCTTATAT
GATGCTGAGACCTTAATGGACAGAATCAAGAAACAGCTACGTGA
ATGGGATGAAAATCTAAAAGATGATTCTCTTCCTTCAAATCCAAT
AGATTTTTCTTACAGAGTAGCTGCTTGTCTTCCTATTGATGATGT
ATTGAGAATTCAGCTCCTTAAAATTGGCAGTGCTATCCAGCGAC
TTCGCTGTGAATTAGACATTATGAATAAATGTACTTCCCTTTGCT
GTAAACAATGTCAAGAAACAGAAATAACAACCAAAAATGAAATAT
TCAGTTTATCCTTATGTGGGCCGATGGCAGCTTATGTGAATCCT
CATGGATATGTGCATGAGACACTTACTGTGTATAAGGCTTGCAA
CTTGAATCTGATAGGCCGGCCTTCTACAGAACACAGCTGGTTTC
CTGGGTATGCCTGGACTGTTGCCCAGTGTAAGATCTGTGCAAG
CCATATTGGATGGAAGTTTACGGCCACCAAAAAAGACATGTCAC
CTCAAAAATTTTGGGGCTTAACGCGATCTGCTCTGTTGCCCACG
ATCCCAGACACTGAAGATGAAATAAGTCCAGACAAAGTAATACT
TTGCTTGACGCGTGGGAGCTCCGGTGGGAGCTCCGGTATCCTC
TGGCATGAGATGTGGCATGAAGGCCTGGAAGAGGCATCTCGTT
TGTACTTTGGGGAAAGGAACGTGAAAGGCATGTTTGAGGTGCT
GGAGCCCTTGCATGCTATGATGGAACGGGGCCCCCAGACTCTG
AAGGAAACATCCTTTAATCAGGCCTATGGTCGAGATTTAATGGA
GGCCCAAGAGTGGTGCAGGAAGTACATGAAATCAGGGAATGTC
AAGGACCTCACCCAAGCCTGGGACCTCTATTATCATGTGTTCCG
ACGAATCTCAAAGTGA
   c. FRB: SEQ ID NO 11 
ATGATCCTCTGGCATGAGATGTGGCATGAAGGCCTGGAAGAGG
CATCTCGTTTGTACTTTGGGGAAAGGAACGTGAAAGGCATGTTT
GAGGTGCTGGAGCCCTTGCATGCTATGATGGAACGGGGCCCC
CAGACTCTGAAGGAAACATCCTTTAATCAGGCCTATGGTCGAGA
TTTAATGGAGGCCCAAGAGTGGTGCAGGAAGTACATGAAATCA
GGGAATGTCAAGGACCTCACCCAAGCCTGGGACCTCTATTATC
ATGTGTTCCGACGAATCTCAAAGTGA
   d. WDR5-NanoLuc^{®}-FKBP12
      WDR5-linker-NanoLuc^{®}-linker-FKBP12: SEQ ID NO 12 
ATGGCGACGGAGGAGAAGCCCGAGACCGAGGCCGCCAGA
GCACAGCCAACCCCTTCGTCATCCGCCACTCAGAGCAAGCCTA
CACCTGTGAAGCCAAACTATGCTCTAAAGTTCACCCTTGCTGGC
CACACCAAAGCAGTGTCCTCCGTGAAATTCAGCCCGAATGGAG
AGTGGCTGGCAAGTTCATCTGCTGATAAACTTATTAAAATTTGG
GGCGCGTATGATGGGAAATTTGAGAAAACCATATCTGGTCACAA
GCTGGGAATATCCGATGTAGCCTGGTCGTCAGATTCTAACCTTC
TTGTTTCTGCCTCAGATGACAAAACCTTGAAGATATGGGACGTG
AGCTCGGGCAAGTGTCTGAAAACCCTGAAGGGACACAGTAATT
ATGTCTTTTGCTGCAACTTCAATCCCCAGTCCAACCTTATTGTCT
CAGGATCCTTTGACGAAAGCGTGAGGATATGGGATGTGAAAAC
AGGGAAGTGCCTCAAGACTTTGCCAGCTCACTCGGATCCAGTC
TCGGCCGTTCATTTTAATCGTGATGGATCCTTGATAGTTTCAAGT
AGCTATGATGGTCTCTGTCGCATCTGGGACACCGCCTCAGGCC
AGTGCCTGAAGACGCTCATCGATGACGACAACCCCCCCGTGTC
TTTTGTGAAGTTCTCCCCGAACGGCAAATACATCCTGGCCGCCA
CGCTGGACAACACTCTGAAGCTCTGGGACTACAGCAAGGGGAA
GTGCCTGAAGACGTACACTGGCCACAAGAATGAGAAATACTGC
ATATTTGCCAATTTCTCTGTTACTGGTGGGAAGTGGATTGTGTCT
GGCTCAGAGGATAACCTTGTTTACATCTGGAACCTTCAGACGAA
AGAGATTGTACAGAAACTACAAGGCCACACAGATGTCGTGATCT
 
CAACAGCTTGTCACCCAACAGAAAACATCATCGCCTCTGCTGCG
CTAGAAAATGACAAAACAATTAAACTGTGGAAGAGTGACTGCGA
ATTCGGGAGCTCCGGTGGGAGCTCCGGTGTCTTCACACTCGAA
GATTTCGTTGGGGACTGGCGACAGACAGCCGGCTACAACCTGG
ACCAAGTCCTTGAACAGGGAGGTGTGTCCAGTTTGTTTCAGAAT
CTCGGGGTGTCCGTAACTCCGATCCAAAGGATTGTCCTGAGCG
GTGAAAATGGGCTGAAGATCGACATCCATGTCATCATCCCGTAT
GAAGGTCTGAGCGGCGACCAAATGGGCCAGATCGAAAAAATTT
TTAAGGTGGTGTACCCTGTGGATGATCATCACTTTAAGGTGATC
CTGCACTATGGCACACTGGTAATCGACGGGGTTACGCCGAACA
TGATCGACTATTTCGGACGGCCGTATGAAGGCATCGCCGTGTT
CGACGGCAAAAAGATCACTGTAACAGGGACCCTGTGGAACGGC
AACAAAATTATCGACGAGCGCCTGATCAACCCCGACGGCTCCC
TGCTGTTCCGAGTAACCATCAACGGAGTGACCGGCTGGCGGCT
GTGCGAACGCATTCTGGCGGGGAGCTCCGGTGGGAGCTCCGG
TACGCGTGGAGTGCAGGTGGAAACCATCTCCCCAGGAGACGG
GCGCACCTTCCCCAAGCGCGGCCAGACCTGCGTGGTGCACTA
CACCGGGATGCTTGAAGATGGAAAGAAATTTGATTCCTCCCGG
GACAGAAACAAGCCCTTTAAGTTTATGCTAGGCAAGCAGGAGGT
GATCCGAGGCTGGGAAGAAGGGGTTGCCCAGATGAGTGTGGG
TCAGAGAGCCAAACTGACTATATCTCCAGATTATGCCTATGGTG
CCACTGGGCACCCAGGCATCATCCCACCACATGCCACTCTCGT
CTTCGATGTGGAGCTTCTAAAACTGGAATGA
xiv. Vector maps for the constructs are given in Figs. 3 to 6. In the vector map given in Fig. 6 NanoLuc^{®} is given as "Luc".

### 3. Transfection and rapamycin treatment

i. After the generation of vectors, HEK293 cells were cultured appropriately prior to assay.
ii. Medium was removed from cell plates by aspiration and cells were trypsinized to dissociate from the cell culture dish bottom.
iii. Trypsin was neutralized using cell culture medium, cells were counted and seeded in the density of 4 million cells into 10 cm dishes in cell culture medium.
iv. The cells were allowed to attach and recover for at least 6 hours at 37 °C, 5% CO₂.
v. The SFFV promoter containing constructs of FKBP12, and FRB were transfected in the weight: weight ratios of 1:1, 1:10 and 1:100(FKBP12 : FRB).
   For each of these transfection ratios the following combinations of plasmid constructs were added in separate tubes to 700 µl of Opti-MEM each:
   a. VHL-FRB (8 µg) and WDR5-NanoLuc^{®}-FKBP12 (0.8 µg)
   b. CRBN-FRB (8 µg) and WDR5-NanoLuc^{®}-FKBP12 (0.8 µg)
   c. FRB (8 µg) and WDR5-NanoLuc^{®}-FKBP12 (0.8 µg)
vi. 30 µl of polyethylenimine (PEI) was added to 700 µl Opti-MEM (per sample), mixed well (vortexed), centrifuged, and incubated at RT for 5 minutes.
vii. The plasmid mixture was added to the PEI mixture, mixed by pipetting up and down and left at RT for 20 min.
viii. The transfection mixture was added to 10 cm plates with attached cells.
ix. The proteins were expressed for at least 18 hours.
x. After 18-24 hours, the cells were resuspended in 12 ml of media after trypsinization.
xi. 40 µl of DMEM was added in all the wells of black 96 well plate and 30 µl of cell suspension per well were seeded into 3x5x2 wells of 96-well plate per transfection condition (for luciferase measurement). The total volume of cell suspension in 96 well plate is 70 µl now. For 96-well plate, 3 wells will be DMSO control, and 3 wells will be treated with 10 nM Rapamycin for 5 different time points (1h, 2h, 4h, 6h and 8h).
   Similarly, 2 x 1ml of cell suspension were seeded into 6-well plates (containing 1 ml media in each well) per transfection condition (for western blot, WB). The total volume of cell suspension in 6-well plate is 2 ml now. For 6-well plate only 6h timepoint was used for treatment.
xii. Plates were shaken back and forth to get even seeding. The plates were left in incubator so that the cells attach overnight.
xiii. Next day, the cells were treated at different time points and harvested together.
xiv. For treatment, 10 nM final concentration of Rapamycin or DMSO was used.
   a. Rapamycin was dissolved in DMSO at a concentration of 10 mM as stock solution. The 10 mM Rapamycin was diluted in DMSO to reach concentration of 10 µM.
   b. For 96 well plate, 2.4 µl DMSO or 10 µM Rapamycin was added in 700 µl warm culture media. 30 µl of the resulting solution was added to the cells in 70 µl media to get the final concentration of 10 nM Rapamycin. The Rapamycin and DMSO solution in media were prepared just before their addition.
   c. For 6-well plate, 2 µl of DMSO or 2 µl 10 µM Rapamycin was added to the cells in 2 ml media to get the final concentration of 10 nM Rapamycin.
xv. For luciferase assay, NanoGlo^{®} Luciferase assay reagent with the substrate (Promega) was added 1:1 with the cells with media, mixed properly, incubated for 5-10 minutes at RT with shaking and luminescence was measured (according to manufacturer's protocol).
xvi. For WB, cells were washed 2 x with ice-cold PBS and harvested in RIPA buffer. BCA assay was performed to determine the protein concentration and the samples were prepared in Laemmli buffer. Equal protein amount per samples were run in PAGE, transferred into PVDF membrane, blocked with 5% milk, and finally probed against WDR5, VHL, CRBN and Vinculin antibodies.

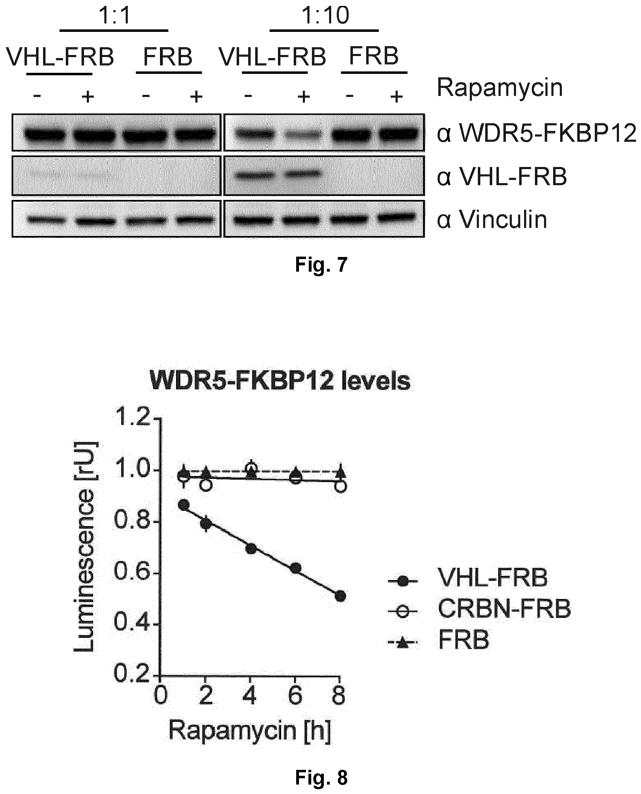

The WB results for WDR5, VHL and Vinculin and transfection ratios of 1:1 and 1:10 (FKBP12 : FRB) are shown in Fig. 7. It has been found that the E3-ligase (FRB) should be in excess to the target protein (FKBP12) counterpart. With the transfection ratio of 1:1 no degradation of WDR5 fusion protein in the presence of Rapamycin was observed in the WB whereas with ratio 1:10 (FKBP12 : FRB) degradation was observed. A similar degradation of WDR5 fusion protein was also observed with a transfection ratio of 1:100 (FKBP12 : FRB). It is assumed that little degradation of WDR5 fusion protein even occurs with the transfection ratio of 1:1 but cannot be observed in WB.

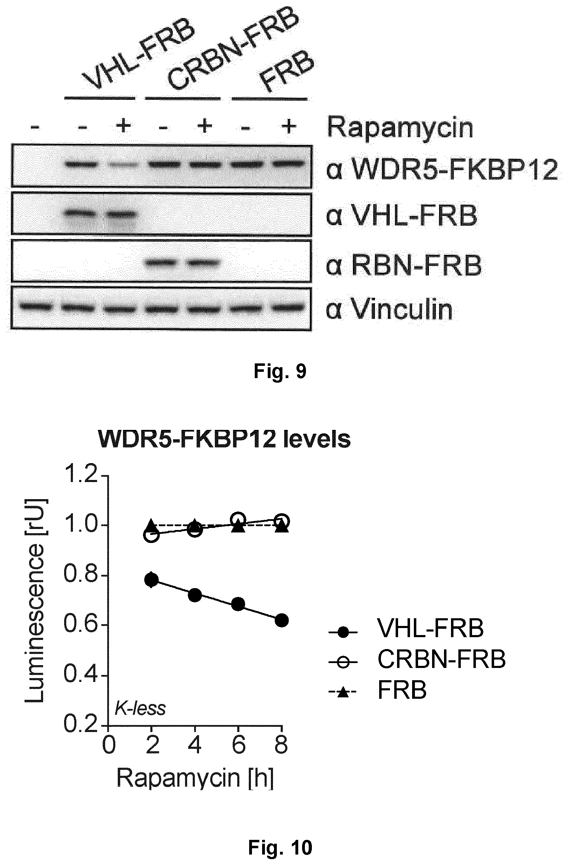

Results of the luciferase assay obtained with a transfection ratio 1:10 (FKBP12 : FRB) are shown in Fig. 8. WB results obtained with a transfection ratio of 1:10 (FKBP12 : FRB) are shown in Fig. 9. The results show the effective degradation of WDR5 by VHL but not by CRBN. This is in line with previous findings obtained with PROTACs indicating that WDR5 can act as a substrate for VHL but not for CRBN.

### 4. Modified system for excluding ubiquitination of the tag

Both FKBP12 and NanoLuc^{®} amino acid sequences comprise 7 and 8 lysine residues (**K**, shown in bold in the sequences below), respectively. Thus, the above assay could not distinguish between ubiquitination of the target protein and of FKBP12 and/or NanoLuc^{®}. For restricting the potential ubiquitination to the target protein the above assay has been modified by replacing the lysine residues by arginine residues.

The canonical sequence of the FKBP12 and NanoLuc^{®} are as follows:
a. FKBP12 (107 amino acids): SEQ ID NO 13 
GVQVETISPGDGRTFPKRGQTCVVHYTGMLEDGKKFDSSRDRNK
PFKFMLGKQEVIRGWEEGVAQMSVGQRAKLTISPDYAYGATGHP
GIIPPHATLVFDVELLKLE
b. NanoLuc^{®} (170 amino acids): SEQ ID NO 1

VFTLEDFVGDWRQTAGYNLDQVLEQGGVSSLFQNLGVSVTPIQRI
VLSGENGLKIDIHVIIPYEGLSGDQMGQIEKIFKVVYPVDDHHFKVIL
HYGTLVIDGVTPNMIDYFGRPYEGIAVFDGKKITVTGTLWNGNKIID
ERLINPDGSLLFRVTINGVTGWRLCERILA

Careful analysis of the publicly available crystal structure of these proteins showed that all the lysine residues are present on the surface of these proteins. Such lysine residues may be potential ubiquitination sites for the E3-ligase in the assay. Such ubiquitination and eventual degradation of the fusion protein might give false positive result in the assay.

Thus, to prevent such false positive results and to improve the assay further, a lysine less (K-less) version of FKBP12 and NanoLuc^{®} construct was generated. For this all the lysine residues from both protein tags, FKBP12 and NanoLuc^{®} were replaced with arginine. Such a K-less construct reliably prevents degradation of target protein only due to ubiquitination of FKBP12 and NanoLuc^{®} sequences in the second recombinant protein. Nucleic acids coding for the K-less versions of FKBP12 and NanoLuc^{®} were ordered as custom double-stranded DNA fragments (gBlock) from the company IDT integrated DNA technologies. The amino acid sequences of the K-less versions of FKBP12 and NanoLuc^{®}, in which replaced arginine residues are shown as **R** in bold, are as follows:
a. FKBP12 (107 amino acids): SEQ ID NO 14 
GVQVETISPGDGRTFPRRGQTCVVHYTGMLEDGRRFDSSRDRNR
PFRFMLGRQEVIRGWEEGVAQMSVGQRARLTISPDYAYGATGHP
GIIPPHATLVFDVELLRLE
b. NanoLuc (170 amino acids): SEQ ID NO 15

VFTLEDFVGDWRQTAGYNLDQVLEQGGVSSLFQNLGVSVTPIQRI
VLSGENGLRIDIHVIIPYEGLSGDQMGQIERIFRVVYPVDDHHFRVIL
HYGTLVIDGVTPNMIDYFGRPYEGIAVFDGRRITVTGTLWNGNRIID
ERLINPDGSLLFRVTINGVTGWRLCERILA

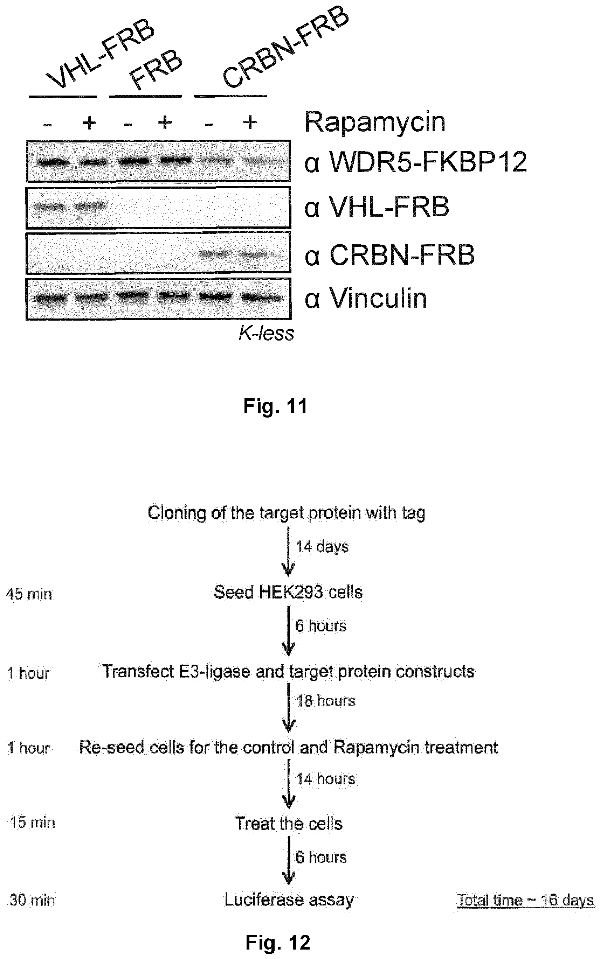

Then the WDR5-NanoLuc-FKBP12 (K-less) construct was generated (similar to the wild-type with the linker in between the proteins), and the assay was repeated as for WDR5-NanoLuc-FKBP12 wild-type with VHL-FRB, CRBN-FRB and FRB. The results from the Nanoluciferase assay and the WB are shown in Fig. 10 and Fig. 11.

Fig. 10 shows WDR5 levels based on luciferase measurement of HEK293 cells transiently expressing WDR5-FKBP12-luciferase (K-less FKBP12 and luciferase) and VHL-FRB or CRBN-FRB or FRB at various timepoints after rapamycin treatment.

Fig. 11 shows an immunoblot of HEK293 cells transiently expressing WDR5-FKBP12-luciferase (K-less FKBP12 and luciferase) and VHL-FRB or CRBN-FRB or FRB, treated with vehicle or rapamycin for 6 hours. Vinculin is used as loading control.

The results show the effective degradation of WDR5 by VHL but not by CRBN. The assay confirms that it was the compatibility of WDR5 with VHL that led to ubiquitination of WDR5 and finally proteasomal degradation of the WDR5-NanoLuc-FKBP12 fusion protein in the first assay and not compatibility of FKBP12 and/or NanoLuc^{®} with VHL.

Once a library of E3-ubiquitin ligases with tags is cloned, a tentative timeline for the assay with any target protein is given in Fig. 12.

## Claims

1. System for checking whether a protein of interest (POI) can act as a substrate for an enzyme selected from an E3-ubiquitin ligase, a SUMO E3 ligase, a deubiquitinating enzyme, a kinase or a phosphatase in a eukaryotic cellular environment, wherein the system comprises
- a first recombinant protein comprising or consisting of an amino acid sequence of the enzyme and an amino acid sequence of a first binding protein of a dimerization system dimerizing the first binding protein with a second binding protein upon addition of a ligand, wherein a first linker is optionally arranged between the amino acid sequence of the enzyme and the amino acid sequence of the first binding protein
or
- a first recombinant nucleic acid coding for the first recombinant protein,
- a second recombinant protein comprising or consisting of
- an amino acid sequence of the POI and an amino acid sequence of the second binding protein of the dimerization system, wherein a second linker is optionally arranged between the amino acid sequence of the POI and the amino acid sequence of the second binding protein, and
- an amino acid sequence of a reporter protein or reporter peptide, wherein a third linker is optionally arranged between the amino acid sequence of the POI and the amino acid sequence of the reporter protein or reporter peptide or between the amino acid sequence of the second binding protein and the amino acid sequence of the reporter protein or reporter peptide,
or
- a second recombinant nucleic acid coding for the second recombinant protein
and
- the ligand.

2. System according to claim 1, wherein the first binding protein of the dimerization system is FKBP-rapamycin binding domain of mTOR complex 1 (FRB) or its analog FRB^{T78L} or another analog of FRB and the second binding protein of the dimerization system is 12-kDa FK506 binding protein (FKBP12) or an analog thereof
or
wherein the first binding protein of the dimerization system is 12-kDa FK506 binding protein (FKBP12) or an analog thereof and the second binding protein of the dimerization system is FKBP-rapamycin binding domain of mTOR complex 1 (FRB) or its analog FRB^{T78L} or another analog of FRB
and
wherein the ligand is rapamycin or an analog of rapamycin which analog is able to dimerize FRB or FRB^{T78L} and FKBP12, wherein the analog is in particular AP21967 in case that the first binding protein or the second binding protein is FRB^{T78L}, or in particular temsirolimus, everolimus, ridaforolimus, umirolimus, or zotarolimus,
wherein any of the before mentioned analogs of FRB and FKBP12 consists of an amino acid sequence having a sequence identity of at least 93 % or a similarity of at least 90 % to the amino acid sequence of FRB and FKBP12, respectively,
wherein the sequence similarity is calculated according to the EMBOSS needle algorithm having an open gap penalty of 10.0 and an extended gap penalty of 0.5 and using the Blosum 62 matrix.

3. System according to claim 2, wherein the sequence identity is at least 95 %, in particular at least 97 % and at most less than 100 % and wherein the sequence similarity is at least 95 %, in particular at least 97 % and at most less than 100 %.

4. System according to any of the preceding claims, wherein in the second recombinant protein
- the reporter protein or reporter peptide and the second binding protein and the second linker and/or third linker in case of presence in the second recombinant protein are chosen such that no lysine residue is present in the amino acid sequences of the reporter protein or reporter peptide, the second binding protein and the second linker and/or third linker
or
- any lysine residue in the amino acid sequence of the reporter protein or reporter peptide, in the amino acid sequence of the second binding protein, in particular of FKBP12 or the analog thereof or of FRB or of its analog FRB^{T78L} or of the other analog of FRB, and in the amino acid sequence of the second linker and/or third linker in case of presence in the second recombinant protein is replaced by an arginine residue or histidine residue, in particular an arginine residue.

5. System according to any of the preceding claims, wherein the system comprises the first recombinant nucleic acid and the second recombinant nucleic acid and the ligand or the first recombinant protein and the second recombinant protein and the ligand.

6. System according to claim 5, wherein the first recombinant nucleic acid is contained in an expression vector, in particular a plasmid or a virus, or a lentiviral vector and the second recombinant nucleic acid is contained in the same or a further expression vector, in particular in particular the plasmid or a further plasmid or the virus or a further virus or the lentiviral vector or a further lentiviral vector, or wherein the first and the second recombinant nucleic acid are contained in the genome of an animal cell, in particular a mammalian cell, after introduction by CRISPR knock-in technique.

7. System according to any of the preceding claims, wherein the reporter protein or reporter peptide is a luciferase, in particular firefly-luciferase, 19 kDa catalytic subunit of Oplophorus luciferase, in particular a luciferase having sequence SEQ ID NO 1, or a luciferase having sequence SEQ ID NO2 (HiBiT), or a fluorophore, in particular green fluorescent protein (GFP), red fluorescent protein (RFP), cyan fluorescent protein(CFP), blue fluorescent protein (BFP), yellow fluorescent protein (YFP), orange fluorescent protein (OFP), far-red fluorescent protein, infra-red fluorescent protein, mNeonGFP (mNG), enhanced GFP (EGFP) or mCherry.

8. System according to any of the preceding claims, wherein the E3-ubiquitin ligase is von Hippel-Lindau E3-ubiquitin ligase (VHL) or cereblon E3-ubiquitin ligase (CRBN) and/or wherein the POI is oncoprotein Myc, oncoprotein MycN , oncoprotein MycL, aurora kinase A (AURKA), aurora kinase B (AURKB), BCR-ABL fusion protein, signal transducer and activator of transcription 3 (STAT3), GTPase Kras (KRAS), EWS-FLI fusion protein, epidermal growth factor receptor (EGFR), Erb-b2 receptor tyrosine kinase 2 (ERBB2), ALK tyrosine kinase receptor (ALK), Wee1-like protein kinase (WEE1), catenin beta-1 (CTNNB1), Yes-associated protein 1 (YAP1), WD repeat-containing protein 5 (WDR5), RuvB-like AAA ATPase 1 (RUVBL1), RuvB-like AAA ATPase 2 (RUVBL2), transcription elongation factor SPT5 (SUPT5H), transcription elongation factor SPT6 (SUPT6H), transcription elongation factor SPT4 (SUPT4H), transformation/transcription domain-associated protein (TRRAP), HECT-UBA and WWE domain containing E3 ubiquitin protein ligase 1 (HUWE1), Bruton tyrosine kinase (BTK), interleukin 1 receptor associated kinase 4 (IRAK4), interleukin 1 receptor associated kinase 3 (IRAK3), mitogen-activated protein kinase kinase kinase kinase 1 (MAP4K1), microtubule associated protein tau (MAPT), mutant huntingtin (mHtt), TAR RNA-binding protein (TARDBP), FIS RNA-binding protein (FUS), glycogen synthase kinase 3 beta (GSK3B), or synuclein alpha (SNCA).

9. Use of the system according to any of the preceding claims for checking whether a protein of interest (POI) can act as a substrate for an enzyme selected from an E3-ubiquitin ligase, a SUMO E3 ligase, a deubiquitinating enzyme, a kinase or a phosphatase, wherein the first recombinant protein, the second recombinant protein and the ligand are brought into contact in an eukaryotic cellular environment or an environment comprising all constituents of an eukaryotic cellular environment or wherein the first recombinant nucleic acid and the second recombinant nucleic acid are co-expressed in eukaryotic cells in cell culture followed by a treatment of the cells with the ligand, wherein a change of cellular concentration of the POI is monitored by measuring the amount or concentration of the reporter protein or reporter peptide in the eukaryotic cells, in the environment comprising all constituents of an eukaryotic cellular environment or in medium in which the eukaryotic cells are suspended.

10. Use according to claim 9, wherein the reporter protein or reporter peptide is a luciferase, wherein its amount or concentration in the cells, in the environment comprising all constituents of an eukaryotic cellular environment or in the medium is measured by measuring of luminescence after conversion of a further substrate by the luciferase, wherein the further substrate is introduced into the cells or added to the environment comprising all constituents of an eukaryotic cellular environment or wherein the cells are lysed and subsequently mixed with the further substrate.

11. Method for checking whether a protein of interest (POI) can act as a substrate for an enzyme selected from an E3-ubiquitin ligase, a SUMO E3 ligase, a deubiquitinating enzyme, a kinase or a phosphatase by use of the system according to any of claims 1 to 8, wherein the method comprises the following steps:
- amplifying nucleic acid fragments coding for the enzyme, for a first binding protein of a dimerization system dimerizing the first binding protein with a second binding protein upon addition of a ligand, for the second binding protein of the dimerization system, for the reporter protein or reporter peptide, for the POI and optionally for the first, the second and/or the third linker by use of polymerase chain reaction (PCR), wherein the reporter protein or reporter peptide is an enzyme,
- cloning the amplified nucleic acid fragments into one or more expression vectors such that the resulting nucleic acid or nucleic acids code for the first recombinant protein and the second recombinant protein,
- transformation of competent bacteria with the vector(s), selecting and expanding bacteria containing the vector(s) and extracting the amplified vector(s),
- culturing eukaryotic cells in cell culture,
- transfection of the vector(s) into the eukaryotic cells,
- expressing the first recombinant protein and the second recombinant protein in the eukaryotic cells,
- treating the eukaryotic cells with the ligand, optionally in combination with a cell membrane penetration promoting substance,
- lysing the eukaryotic cells, adding a further substrate for conversion by the reporter protein or reporter peptide and (an) additional substance(s) in case the additional substance(s) is/are required for a conversion of the further substrate by the reporter protein or reporter peptide and determining whether a conversion of the further substrate changes over time, wherein a change of the conversion is an indication for the acting of the POI as a substrate for the enzyme.

12. Method according to claim 11, wherein the eukaryotic cells are mammalian cells, in particular HEK293 cells and/or wherein the first binding protein of the dimerization system is FKBP-rapamycin binding domain of mTOR complex 1 (FRB) or its analog FRB^{T78L} or another analog of FRB, the second binding protein of the dimerization system is 12-kDa FK506 binding protein (FKBP12) or an analog thereof and the ligand is rapamycin or an analog of rapamycin able to dimerize FRB or FRB^{T78L} and FKBP12, in particular AP21967 in case that the first binding protein is FRB^{T78L}, temsirolimus, everolimus, ridaforolimus, umirolimus, or zotarolimus, wherein any of the before mentioned analogs of FRB and FKBP12 consists of an amino acid sequence having a sequence identity of at least 93 % or a similarity of at least 90 % to the amino acid sequence of FRB and FKBP12, respectively, wherein the sequence similarity is calculated according to the EMBOSS needle algorithm having an open gap penalty of 10.0, an extended gap penalty of 0.5 and using the Blosum 62 matrix.

13. Method according to claim 11 or 12, wherein the E3-ubiquitin ligase is von Hippel-Lindau E3-ubiquitin ligase (VHL) or cereblon E3-ubiquitin ligase (CRBN) and/or wherein the POI is oncoprotein Myc, oncoprotein MycN , oncoprotein MycL, aurora kinase A (AURKA), aurora kinase B (AURKB), BCR-ABL fusion protein, signal transducer and activator of transcription 3 (STAT3), GTPase Kras (KRAS), EWS-FLI fusion protein, epidermal growth factor receptor (EGFR), Erb-b2 receptor tyrosine kinase 2 (ERBB2), ALK tyrosine kinase receptor (ALK), Wee1-like protein kinase (WEE1), catenin beta-1 (CTNNB1), Yes-associated protein 1 (YAP1), WD repeat-containing protein 5 (WDR5), RuvB-like AAA ATPase 1 (RUVBL1), RuvB-like AAA ATPase 2 (RUVBL2), transcription elongation factor SPT5 (SUPT5H), transcription elongation factor SPT6 (SUPT6H), transcription elongation factor SPT4 (SUPT4H), transformation/transcription domain-associated protein (TRRAP), HECT-UBA and WWE domain containing E3 ubiquitin protein ligase 1 (HUWE1), Bruton tyrosine kinase (BTK), interleukin 1 receptor associated kinase 4 (IRAK4), interleukin 1 receptor associated kinase 3 (IRAK3), mitogen-activated protein kinase kinase kinase kinase 1 (MAP4K1), microtubule associated protein tau (MAPT), mutant huntingtin (mHtt), TAR RNA-binding protein (TARDBP), FIS RNA-binding protein (FUS), glycogen synthase kinase 3 beta (GSK3B), or synuclein alpha (SNCA).

14. Method according to any of claims 11 to 13, wherein the reporter protein or reporter peptide is a luciferase, in particular firefly-luciferase, 19 kDa catalytic subunit of Oplophorus luciferase, in particular a luciferase having sequence SEQ ID NO 1, or a luciferase having sequence SEQ ID NO2 (HiBiT), or a fluorophore, in particular green fluorescent protein (GFP), red fluorescent protein (RFP), cyan fluorescent protein(CFP), blue fluorescent protein (BFP), yellow fluorescent protein (YFP), orange fluorescent protein (OFP), far-red fluorescent protein, infra-red fluorescent protein, mNeonGFP (mNG), enhanced GFP (EGFP) or mCherry.

15. Method according to any of claims 11 to 15, wherein the cell membrane penetration promoting substance is dimethyl sulfoxide (DMSO).
